# EUROPEAN PATENT APPLICATION

(11) **EP 4 016 539 A1**
(43) Date of publication of application: **22.06.2022**
(21) Application number: 20214288.1
(22) Date of filing: 15.12.2020
(51) Int. Cl.: G16H 20/70, A61B 5/16, G16H 40/20

(54) **NEURAL ACTIVITY CONTROLLED VIRTUAL REALITY SYSTEM**

(71) Applicant: Koninklijke Philips N.V., 5656 AG Eindhoven (NL)
(72) Inventor: VAN DOOREN, Marieke, 5656 AE Eindhoven (NL); LEUFKENS, Timmy Robertus Maria, 5656 AE Eindhoven (NL); VAN EE, Raymond, 5656 AE Eindhoven (NL); JOHNSON, Mark, 5656 AE Eindhoven (NL); MENA BENITO, Maria Estrella, 5656 AE Eindhoven (NL); HUIJBERS, Willem, 5656 AE Eindhoven (NL)
(74) Representative: Philips Intellectual Property & Standards

(57) **Abstract**

Disclosed herein is a medical instrument (100, 300) comprising an activity measurement system (110, 301, 302) configured for measuring brain activity data (136) from a subject (102), a memory (120) storing machine executable instructions (130) and storing a simulation model (132), wherein the simulation model is configured to generating simulation data (134), wherein the simulation model is configured for modifying the simulation data using a model parameter (140), a virtual reality system (106, 106', 106", 106"', 106"") configured for rendering the simulation data; and a computational system (114) for controlling the medical instrument. Execution of the machine executable instructions causes the computational system to set (200) the model parameter to an initial value (142). Execution of the machine executable instructions further causes the computational system to repeatedly: generate (202) the simulation data using the simulation model; render (204) the simulation data using the virtual reality system; measure (206) the brain activity data using the activity measurement system; calculate (208) a neural activity metric (138) descriptive of neural activity of the subject from the brain activity data; increase (210) the model parameter to adjust the simulation model if the neural activity metric is below a first predetermined value (144); and decrease (212) the model parameter to adjust the simulation model if the neural activity metric is above a second predetermined value (146).

## Description

### FIELD OF THE INVENTON

The invention relates to magnetic resonance imaging, in particular to controlling a virtual reality system using measured brain activity.

### BACKGROUND OF THE INVENTION

Various measurement modalities may be used to measure brain activity of a subject. Functional magnetic resonance imaging is one example of a means of directly measuring which portions of a subject's brain is active. A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. Many functional magnetic resonance imaging (fMRI) techniques measure brain activity directly by detecting changes in blood flow within the brain.

European patent application publication EP3659499A discloses a medical instrument comprising an activity measurement system configured for measuring brain activity data from a subject. The medical instrument further comprises a stimulus presentation system configured for providing sensory stimulus to the subject. The medical instrument further comprises a memory for storing machine executable instructions and for storing a stimulus reinforcer database. The stimulus reinforcer database comprises entries. Each entry comprises commands configured for controlling the stimulus presentation system to provide the sensory stimulus to the subject. The medical instrument further comprises a processor for controlling the medical instrument. Execution of the machine executable instructions causes the processor to: control the stimulus presentation system with a set of entries selected from the stimulus reinforcer database to repeatedly provide sensory stimulus to the subject; control the activity measurement system for performing the measurement of the brain activity data during each sensor stimulus; select a chosen entry from the set of entries using the brain activity data; and store the chosen entry in the memory.

### SUMMARY OF THE INVENTION

The invention provides for a medical instrument, a computer program, and a method in the independent claims. Embodiments are given in the dependent claims.

Many types of therapy rely on a psychologist or therapist providing sensory stimulation to a subject and then adjusting the stimulation to the subject based on subjective observation of the subject. Embodiments may provide a medical instrument or tool which may be used to better evaluate and control the sensory stimulation provided to a subject. This may be achieved by measuring brain activity data with an activity measurement system. The brain activity data is then used to calculate a neural activity metric. The neural activity metric is then used to modify or control a model parameter. Adjustment of the model parameter changes simulation data produced by a simulation model. The virtual reality system then renders the model data. As the subject is exposed to the stimuli provided by the virtual reality system, the brain activity data is reacquired and the process continues as a closed loop. Embodiments adjust the model parameter to keep the neural activity metric between a first predetermined value and a second predetermined value. For example, as a subject becomes adapted to stimulus provided by the virtual reality system the medical instrument may adjust the model parameter.

In one aspect the invention provides for a medical instrument that comprises an activity measurement system configured for measuring brain activity data from a subject. The activity measurement system may take different forms in different examples. For example, there may be activity measurement systems which measure the functional activity of the brain such as functional magnetic resonance imaging. In other instances, the activity measurement system may comprise electrical measurements such as an EEG system.

The medical instrument further comprises a memory storing machine-executable instructions and storing a simulation model. The simulation model is configured to generate simulation data. The simulation model is also configured for modifying the simulation data using a model parameter. The simulation data may be for providing data for a virtual reality system. The medical instrument further comprises a virtual reality system that is configured for rendering the simulation data. The virtual reality system may take different forms in different examples. The virtual reality system may be configured for providing visual, tactile, temperature, pressure and/or audible sense stimulation to a subject. The rendering of the simulation data configures or controls the virtual reality system to provide this sensory stimulus to the subject.

The medical instrument further comprises a computational system for controlling the medical instrument. Execution of the machine-executable instructions causes the computational system to set the model parameters to an initial value. This initial value may be a global value in some cases and in other cases it may be specific or loaded for a particular subject. This initial value may for example put the simulation model into an initial state for starting the method.

Execution of the machine-executable instructions further causes the computational system to repeatedly generate the simulation data using the simulation model. During the first loop the simulation model is controlled with the model parameter set to the initial value. In other loops the model parameter is updated. Execution of the machine-executable instructions further causes the computational system to repeatedly render the simulation data using the virtual reality system. This may be performed by inputting the simulation data into the virtual reality system. Execution of the machine-executable instructions further causes the computational system to repeatedly measure the brain activity data using the activity measurement system.

Execution of the machine-executable instructions further causes the computational system to repeatedly calculate a neural activity metric description of the neural activity of the subject from the brain activity data. The neural activity metric may for example be one or more numerical values which are used to describe or characterize the neural activity of the subject. Execution of the machine-executable instructions further causes the computational system to repeatedly increase the model parameter to adjust the simulation model if the neural activity metric is below a first predetermined value and also to decrease the model parameter to adjust the simulation model if the neural activity is above a second predetermined value. The goal of the system is therefore to maintain the neural activity metric between the first predetermined value and the second predetermined value.

In some examples, the model parameter could have one or more values or multiple values which are input into the simulation model. This single value or vector of values could be used to control or modify the behavior of the simulation model which in turn causes changes in the simulation data and what the subject experiences via the virtual reality system. This embodiment may be beneficial because it provides for a closed feedback loop which may be used to control the neural activity metric. This may be used to maintain the neural activity metric between the set values represented by the first predetermined value and the second predetermined value. This for example may be useful in various types of psychological therapy.

In another embodiment the brain activity measurement system comprises a magnetic resonance imaging system. The memory further contains pulse sequence commands configured for acquiring k-space data from an excitation region of interest of the subject according to a functional magnetic resonance imaging protocol. When magnetic resonance imaging is performed the magnetic resonance imaging system is controlled with pulse sequence commands such that a region of the subject is excited or has the spins excited and then data from this excitation region of interest is then read. The excitation region of interest is therefore equivalent with the region of the subject which is actively being imaged.

Functional magnetic resonance imaging protocol is a magnetic resonance imaging protocol which can be used to measure typical responses of a subject when brain or neurological tissue is currently being used. For example, functional magnetic resonance imaging can be used to determine which portions of a person's brain are active when the subject is undergoing stimulus.

The measuring of the brain activity data using the activity measurement system comprises controlling the magnetic resonance imaging system to acquire the k-space data. This embodiment may be beneficial because the functional magnetic resonance imaging is able to directly measure which portions of the brain of the subject are currently active. This may be useful in measuring directly how a subject is responding to the stimulus provided by the virtual reality system. This may enable the measurement of the neural activity metric objectively and without input from a psychologist or other healthcare professional or the subject themselves. The subject's response to the stimulus provided by the virtual reality system is measured directly.

In another embodiment the magnetic resonance imaging protocol is a blood oxygenation level dependent functional magnetic resonance imaging protocol. This protocol is configured for measuring neural activity within one or more volumes selected within the excitation region of interest. The one or more volumes are a region or sub-regions of the excitation region of interest. For example, when a subject is experiencing fear or some stimulus different portions of the subject's brain may become active. There may be advantages in measuring multiple locations and correlating their behavior to determine the neural activity metric.

In another embodiment the one or more volumes may comprise the amygdala.

In another embodiment the one or more volumes may comprise the ventromedial prefrontal cortex.

In another embodiment the one or more volumes comprises the frontal parietal network. During sustained periods of a demanding cognitive workload, entailing cognitive fatigue, humans typically display time-on-task (TOT) effects, in which performance gets steadily worse over the period of task engagement. There may be persistent effects of cognitive fatigue in the frontal-parietal network after a period of heavy mental work and indicate the critical role of this attentional network in mediating TOT effects. Such a cognitive fatigue task also leads to cumulative cortisol levels.

In another embodiment the one or more volumes comprises the hippocampus. This for example may be useful in treating Post Traumatic Stress Disorder (PTSD) patients. In PTSD patients, the volume of the hippocampus (which is associated with placing memories in the correct context of space and time) is significantly reduced.

In another embodiment the one or more volumes comprises in the default mode network. A study involving fMRI data from 848 patients with Major Depressive Disorder (MDD) and 794 normal controls reported decreased functional connectivity in the default mode network in recurrent MDD, confirming a key role of the default mode network in MDD. The decreased functional connectivity in the default mode network was positively related to symptom severity in recurrent MDD.

In another embodiment the one or more volumes comprises both the amygdala and ventromedial prefrontal cortex. Spontaneous neuronal activity following a fear (such as claustrophobia) memory activation. Significant enhanced functional connectivity between the amygdala and ventromedial prefrontal cortex upon fear memory activation group.

In another embodiment the activity measurement system comprises a magnetoencephalography system.

In another embodiment the activity measurement system comprises an electroencephalography system.

In another embodiment the activity measurement system comprises an optically pumped magnetometer magnetoencephalography brain measurement system.

In another embodiment the activity measurement system comprises a functional near-infra-red spectroscopy system.

In another embodiment the activity measurement system comprises an eye dilation measurement system.

In another embodiment the activity measurement system comprises a heart rate measurement system.

In another embodiment the activity measurement system comprises a respiration rate system.

In another embodiment the activity measurement system comprises a subject motion detection system.

Some of the above are not necessarily measurements that are performed directly on the brain or brain of the subject. However, such things as eye dilation, heart rate, respiration and involuntary motion of the subject may also be included when calculating the activity metric.

In another embodiment the memory further contains a control algorithm configured for outputting the model parameter in response to receiving the neural activity metric, the first predetermined value, and the second predetermined value. Execution of the machine-executable instructions further causes the computational system to determine the model parameter by inputting the neural activity metric, the first predetermined value, and the second predetermined value into the control algorithm. This embodiment may be beneficial because sophisticated control algorithms can be used for providing the feedback loop and controlling the neural activity metric.

In another embodiment the control algorithm is implemented as a lookup table.

In another embodiment the control algorithm is implemented as a multi-dimensional lookup table.

In another embodiment the control algorithm is implemented as a neural network. The medical instrument and measurement of the neural activity metric from many subjects as a function of the model parameter can be used as data to be used to train the neural network.

In another embodiment the control algorithm is implemented as a feedback loop. For example, the control algorithm could implement a negative feedback control system. In other examples, the control algorithm implements a proportional-integral-derivative controller or a closed-loop transfer function.

In another embodiment the virtual reality system comprises a visual display.

In another embodiment the virtual reality system comprises a three-dimensional display. This may for example be a flat three-dimensional display or other system which provides images to each eye of the subject.

In another embodiment the virtual reality system comprises a virtual reality headset or an augmented reality headset. This may be beneficial because it may provide lifelike or realistic stimulus to the subject.

In another embodiment the virtual reality system comprises a headphone or an audio speaker, or subwoofer. Each of these may be beneficial because they may provide audio stimulus to the subject.

In another embodiment the virtual reality system comprises a tactile feedback system which may for example be used to simulate the interaction of a subject with a stimulus.

In another embodiment the virtual reality system comprises a heater.

In another embodiment the virtual reality system comprises a cooler.

In another embodiment the virtual reality system further comprises an instruction display for providing manual tactile feedback instructions. For example, an assistant or helper may look at the instruction display and then provide the tactile feedback manually to the subject.

In another embodiment the virtual reality system further comprises an olfactory stimulus generator which may for example be used to emulate smells that the subject smells.

In another embodiment the virtual reality system is configured for rendering multiple sensory stimuli using rhythmically synchronous stimulation. In this example the multiple sensory stimulus are provided in a manner which are synchronized to each other. This may have the benefit of enhancing the believability of the simulation as well as enhancing the psychological response.

In another embodiment the simulation model is configured such that an increase in the model parameter induces an initial increase in the neural activity metric. For example, when a scary image is moved closer to the subject or modified the subject may initially experience an increase in anxiety or activity in the portions of the brain which increase when the subject does experience anxiety.

In another embodiment the decrease of the model parameter if the neural activity metric is above the second predetermined value is a function of the velocity of the neural activity metric when it exceeds the second predetermined value. In some types of neural stimulus if the subject is over-stimulated the stimulus may be counterproductive. Therefore, going over the second predetermined value slowly may have a different function or effect on the psychology of the subject than going quickly. If the neural activity metric goes over the second predetermined value too quickly it may for example be beneficial to reduce the model parameter to a lower value than if it was exceeded slowly.

In another embodiment the decrease of the model parameter decreases as the velocity of the neural activity metric increases when it exceeds the second parameter value. This embodiment may be beneficial for example to help prevent over-stimulation of the subj ect.

In another aspect the invention provides for a computer program comprising machine-executable instructions for execution by a computational system that controls a medical instrument. The medical instrument comprises an activity measurement system that is configured for measuring brain activity data from a subject. The medical instrument further comprises a virtual reality system that is configured for rendering simulation data or providing simulation data to the subject. The medical instrument further comprises a memory storing a simulation model. The simulation model is configured to generate simulation data.

The simulation model is configured for modifying the simulation data using a model parameter. Execution of the machine-executable instructions causes the computational system to set the model parameter to an initial value. Execution of the machine-executable instructions further causes the computational system to repeatedly generate the simulation data using the simulation model. Execution of the machine-executable instructions further causes the computational system to repeatedly render simulation data using the virtual reality system. Execution of the machine-executable instructions further causes the computational system to repeatedly measure the brain activity data using the activity measurement system.

Execution of the machine-executable instructions further causes the computational system to repeatedly calculate a neural activity metric descriptive of neural activity of the subject from the brain activity data. Execution of the machine-executable instructions further causes the computational system to repeatedly increase the model parameter to adjust the simulation model if the neural activity metric is below a first predetermined value. Execution of the machine-executable instructions further causes the computational system to repeatedly decrease the model parameter to adjust the simulation model if the neural activity metric is above a second predetermined value.

In another aspect the invention provides for a method of controlling a medical instrument. The medical instrument comprises an activity measurement system configured for measuring brain activity data from a subject. The medical instrument further comprises a virtual reality system configured for rendering simulation data to the subject. The medical instrument further comprises a memory storing a simulation model. The simulation model is configured for generating simulation data. The simulation model is configured for modifying the simulation data using a model parameter.

The method comprises setting the model parameter to an initial value. The method further comprises repeatedly generating the simulation data using the simulation model. The method further comprises repeatedly rendering the simulation data using the virtual reality system. The method further comprises repeatedly measuring the brain activity data using the activity measurement system. The method further comprises repeatedly calculating a neural activity metric descriptive of neural activity of the subject from the brain activity data. The method further comprises repeatedly increasing the model parameter to adjust the simulation model if the neural activity metric is below a first predetermined value. The method further comprises repeatedly decreasing the model parameter to adjust the simulation model if the neural activity metric is above a second predetermined value.

In another embodiment the method further comprises selecting the simulation model such that rendering the simulation data using the virtual reality system evokes an emotional or psychological response from the subject that is initially increased by an increase in the model parameter. The simulation model may for example be selected by having the subject fill out a questionnaire. In other examples the simulation model may be provided by a healthcare provider. In yet other examples the simulation model may be selected by exposing the subject to a library containing different simulation models and it may be measured using the activity system to determine which of these models evokes a larger emotional response.

In another embodiment the emotional or psychological response is fear.

In another embodiment the emotional or psychological response is anxiety.

In another embodiment the emotional or psychological response is stress.

In another embodiment the emotional or psychological response is depression.

In another embodiment the emotional or psychological response is pain.

In another embodiment the emotional or psychological response comprises addictive thoughts.

In another embodiment the emotional or psychological response comprises maladaptive thoughts.

In another embodiment the emotional or psychological response comprises obsessive-compulsive response or thoughts.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

An activity measurement system as used herein is an instrument which is able to measure brain activity data. Brain activity data is data which is descriptive of neural activity of a subject.

A virtual reality (VR) system as used herein is a device which is able to simulate sensory stimulation for a subject. A VR system often times includes a three-dimensional display or virtual reality glasses, but may include or be formed from other equipment which can provide sensory stimulation such as a tactile feedback system or generator, a temperature control system such as a heater or cooler, a sound system such as a headphone and or speaker system, and/or an olfactory

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image or MR image is defined herein as being the reconstructed two- or three-dimensional visualization of anatomic data contained within the k-space data. This visualization can be performed using a computer. A functional magnetic resonance image is a magnetic resonance image which contains a mapping descriptive of spatially dependent neural or brain activity.

### BRIEF DESCRIPTION OF THE DRAWINGS

In the following preferred embodiments of the invention will be described, by way of example only, and with reference to the drawings in which:
Fig. 1 illustrates an example of a medical instrument;
Fig. 2 shows a flow chart which illustrates a method of operating the medical instrument of Fig.;
Fig. 3 illustrates a further example of a medical instrument;
Fig. 4 illustrates an example of a rendering of simulation data;
Fig. 5 illustrates an example of a rendering of simulation data;
Fig. 6 illustrates an example of a rendering of simulation data; and
Fig. 7 illustrates an example of a rendering of simulation data.

### DESCRIPTION OF EMBODIMENTS

Like numbered elements in these figures are either equivalent elements or perform the same function. Elements which have been discussed previously will not necessarily be discussed in later figures if the function is equivalent.

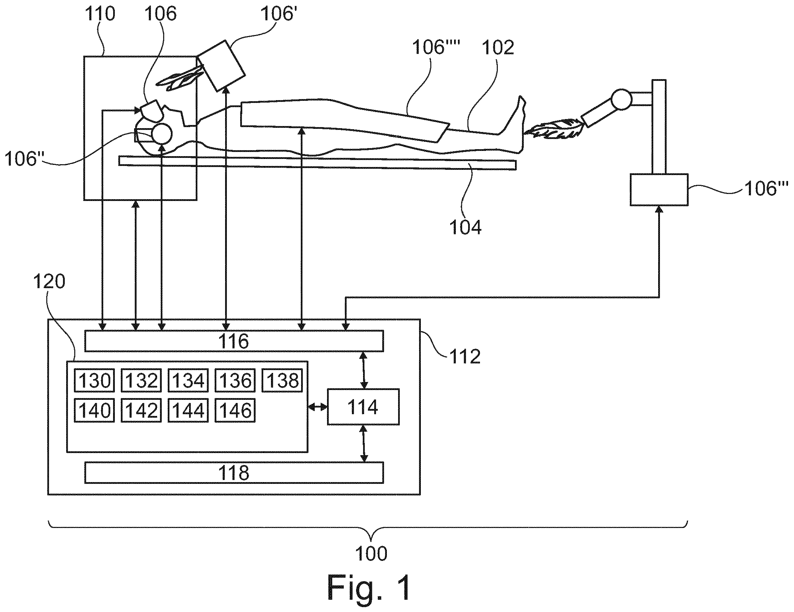

Fig. 1 illustrates an example of a medical instrument 100. A subject 102 is shown as reposing on a subject support 104 as using the medical instrument 100. The medical instrument 100 comprises a virtual reality system. The virtual reality system can comprise of different components. In one example the virtual reality system comprises virtual reality glasses 106. The virtual reality system may further comprise an olfactory stimulus generator 106'. The olfactory stimulus generator may release an aerosol or other amount of a stored substance or substances that stimulate the olfactory sense of the subject.

The virtual reality system may further comprise headphones 106" or other system for providing audio signals to the subject 102. The virtual reality system may further comprise a tactile feedback system 106"'. The virtual reality system may further comprise a temperature control system 106"" that for example could be a heater and/or cooling system for heating or cooling portions of the body of the subject 102. The virtual reality system can be made up of any one or a combination of these components.

The medical instrument 100 is further shown as comprising an activity measurement system 110 that is configured for measuring brain activity data of the subject 102. The activity measurement system 110 here is representative. It for example may be a magnetic resonance imaging system, a magnetoencephalography system, an electroencephalography system, an optically pumped magnetometer magnetoencephalography brain measurement system, a functional near-infrared spectroscopy system, an eye dilation measurement system, a heart rate measurement system, a respiration rate system, a subject motion detection system, and combinations thereof

The various components of the virtual reality system and the activity measurement system 110 are shown as being connected to a hardware interface 116 of a computer system 112. The computer 112 comprises a computational system 114. The computational system 114 is intended to represent one or more computational systems or processing cores that may be located at one or more locations. The computational system 114 is shown as being connected to the hardware interface 116. The hardware interface 116 allows the computational system 114 to exchange data with as well as control other components of the medical instrument 100. The computational system 114 is also shown as being connected to an optional user interface 118 and a memory 120. The memory 120 is intended to represent any type of memory that is accessible to the computational system 114.

The memory 120 is shown as containing machine-executable instructions 130. The machine-executable instructions 130 enable the computational system 114 to perform various tasks such as controlling other components as well as performing data and image processing tasks. The memory 120 is further shown as containing a simulation model 132. The simulation model 132 is configured for generating simulation data 134. The simulation data 134 as used herein encompasses data which can be used to control the various components of the virtual reality system to provide sensory stimulus to the subject 102 to provide a virtual environment. The memory 120 is shown as containing simulation data 134 generated by the simulation model 132.

The memory 120 is further shown as containing brain activity data 136 measured by the activity measurement system 110. The memory 120 is further shown as containing a neural activity metric 138 that has been calculated from the brain activity data 136. The memory 120 for example may contain an algorithm which calculates one or more numerical values from the brain activity data 136 and this may be the neural activity metric 138. The memory 120 is further shown as containing a model parameter 140. The model parameter 140 is a parameter used to control the simulation model 132. By changing the model parameter 140 the content which the simulation model 132 outputs in the form of simulation data 134 may be changed. The memory 120 is further shown as containing an initial value 142. This may be an initial setting if the model parameter 140 is set when the subject 102 begins to use the medical instrument 100. The memory 120 is further shown as containing a first predetermined value 144 and a second predetermined value 146. The model parameter 140 is adjusted so that the neural activity metric 138 stays between the first predetermined value 144 and the second predetermined value 146. Various control algorithms can be used to perform this.

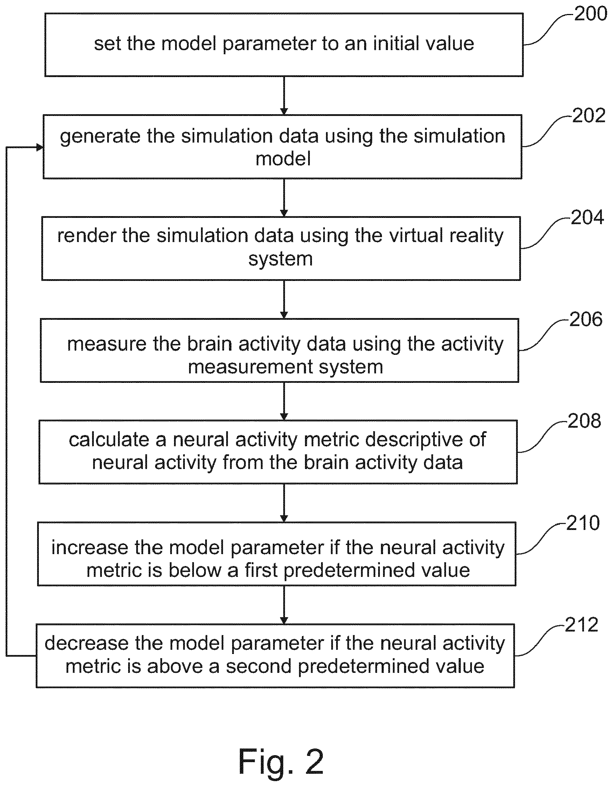

Fig. 2 shows a flowchart which illustrates a method of using the medical instrument 100 of Fig. 1. First, in step 200, the model parameter 140 is set to the initial value 142. Next, in step 202, the simulation data 134 is generated with the simulation model 132. In step 204, the simulation data 134 is rendered with the components of the virtual reality system 106, 106', 106", 106"', 106"". Then, in step 206, the brain activity data 136 is measured using the activity measurement system 110. Next, in step 208, the neural activity metric 138 is calculated from the brain activity data 136. Then, in step 210, the model parameter is increased if the neural activity metric 138 is below the first predetermined value 144. Next, in step 212, the model parameter 140 is decreased if the neural activity metric 138 is above a second predetermined value 146. After step 212 is finished, the method may then return back to step 202 and this may be completed multiple times as a closed control loop.

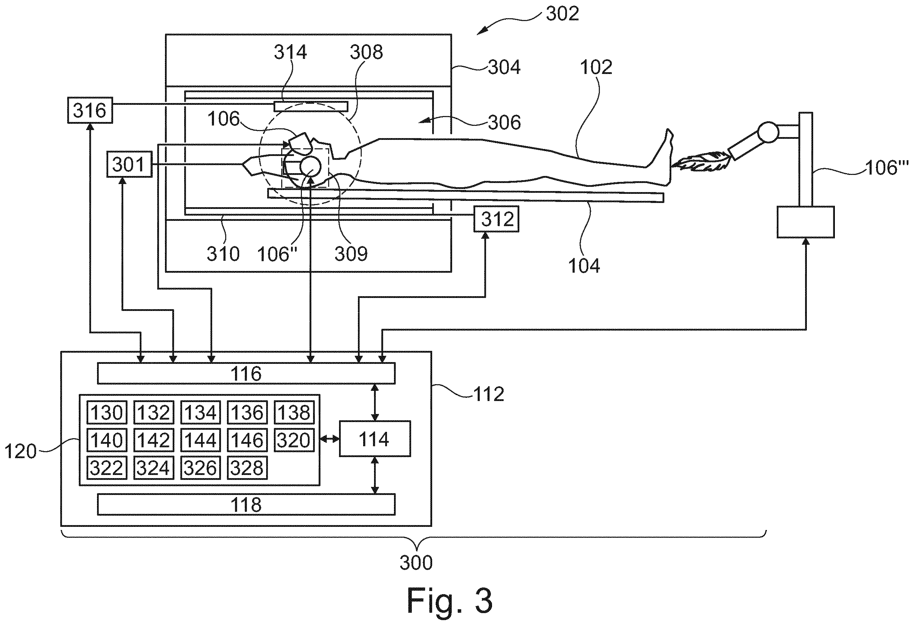

Fig. 3 illustrates a further example of a medical instrument 300. The medical instrument 300 is similar to that depicted in Fig. 1 except it is shown as comprising a magnetic resonance imaging system 302 and an optional EEG system 301. Both the magnetic resonance imaging system 302 and, in some cases, the optional EEG system 301 can be used to provide the brain activity data 136.

The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. An excitation region of interest 309 is shown within the imaging zone 308. The magnetic resonance data that is acquired typically acquried for the excitation region of interest. A subject 102 is shown as being supported by a subject support 104 such that a brain region of the subject 102 is within the imaging zone 308 and the excitation region of interest 309. The EEG system 301 is shown as having electrodes going to the head area of the subject 102.

Within the bore 306 of the magnet there is also a set of magnetic field gradient coils 310 which is used for acquisition of preliminary magnetic resonance data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receivers. The radio-frequency coil 314 may also have multiple receive/transmit elements and the radio frequency transceiver 316 may have multiple receive/transmit channels. For example if a parallel imaging technique such as SENSE is performed, the radio-frequency could 314 will have multiple coil elements.

The transceiver 316 and the gradient controller 312 are shown as being connected to the hardware interface 116 of the computer 112.

The memory 120 is further shown as storing pulse sequence commands 320. The pulse sequence commands 320 may for example be used for measuring k-space data 322 according to a functional magnetic resonance imaging protocol. A functional magnetic resonance imaging protocol acquires data which can be used to infer where brain activity is occurring within the brain of the subject 102. The memory 120 is further shown as containing k-space data 322 that was acquired by controlling the magnetic resonance imaging system 302 with the pulse sequence commands 320. The memory 120 is further shown as containing functional magnetic resonance image data that was provided by reconstructing it from the k-space data 322. The memory 120 is also shown as optionally containing EEG data 326 that was acquired with the EEG system 301. The functional magnetic resonance image 324 and sometimes also the optional EEG data 326 may be used to provide the brain activity data 136.

The memory is also shown as containing an optional control algorithm 328 which may be used to set the value of the model parameter after each iterative loop. This may for example be implemented as a feedback loop or a neural network or other control algorithm.

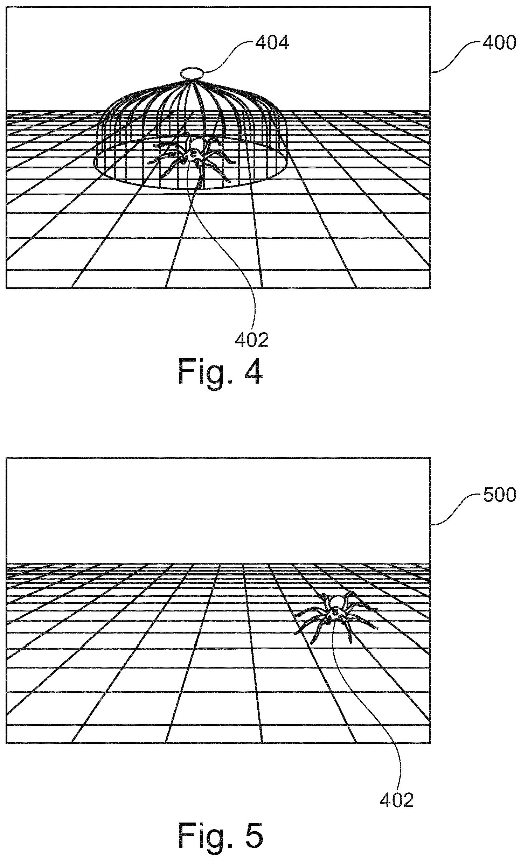

Figs. 4-7 are used to illustrate how the rendering of the simulation data 134 can change as the model parameter 140 is varied. Fig. 4 depicts a representation of a three-dimensional image 400. Depicted in this is a spider 402 that is far away and in a cage 404. This for example, may be an illustration of an initial value 142. The spider in this image is non-threatening. This may for example produce only minimal amounts of anxiety in a person who is afraid of spiders. The simulation could include the spider 402 moving within the cage.

As the model parameter is changed Fig. 5 represents a change in the three-dimensional image 500. The spider 402 is still far away but is no longer within the cage 404. As the subject becomes adapted to this, the brain activity data 136 will decrease as will the neural activity metric 138. The model parameter 140 is then changed to induce at least a temporary increase in the neural activity metric 138. The simulation could for example have the spider move at a distance far from the subject.

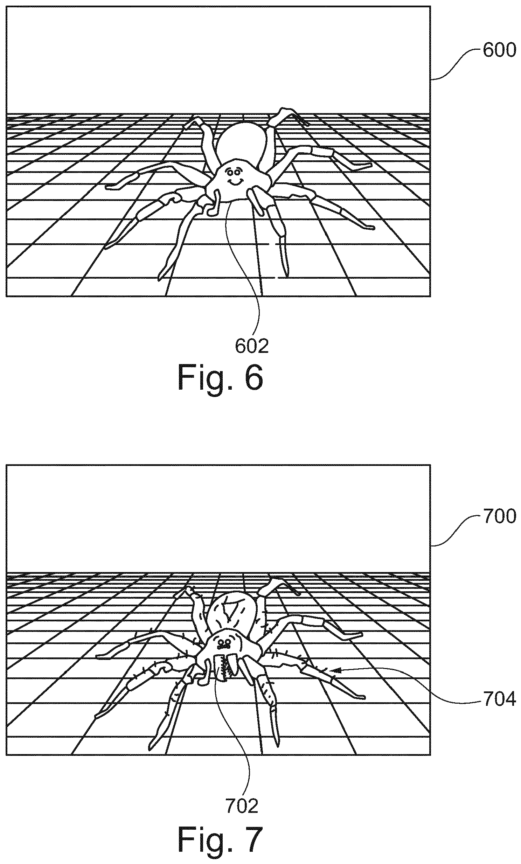

The model parameter 140 has been increased which has caused the spider to move closer. Fig. 6 shows a three-dimensional image with a spider 602 that is closer. The spider depicted has a more friendly and anthropologic appearance. The spider only has two eyes, lacks hair on its legs and body, and has a smile instead of fangs.

As the subject becomes more adapted to this three-dimensional image or virtual reality simulation the model parameter is changed again. Fig. 7 shows a further three-dimensional image 700, an additional view of the spider 702. The spider 702 has a less friendly or scarier appearance and has various features which are modified. For example, the spider 702 in this example has additional leg hairs 704. As the model parameter 140 is changed the simulation morphs between the various images depicted in Figs. 4-7. By changing this in degrees the subject may become more adapted to and eventually lose his or her fear of spiders.

Real-time fMRI brain signals can be used as a tool to adjust virtual-reality content, for example during therapy. Existing adjustment methods consist merely of choosing an image or piece of music from a limited number of predefined fixed images and pieces of music.

Therapy literature demonstrates that transfer of learned skills from the therapy environment to the real world are biggest when the stimulation content is immersive, and specifically personalized to the relevance for an individual patient (subject 102).

Examples may overcome the shortcomings of the current approaches by providing an optimized virtual reality therapy according to the personal needs of the patient. It comprises a system and method for close-loop personalization of immersive virtual reality environment therapy. Personalized virtual reality therapy is provided by a stimulation system that dynamically adapt parametrically one or more variables in a feature table (like e.g., the proximity, size, ... of a fear object) according with the real time measurements of based on the real time brain activity measurements (e.g. real-time fMRI, EEG, fNIRS, ...).

Virtual reality approaches have been demonstrated to be beneficial in many aspects of psychiatry and mental disorders such as anxiety disorders, substance abuse, obsessive compulsive disorders etc. It may be started by considering anxiety disorders as a carrier to explain examples.

Anxiety disorders can cause significant fear in certain contexts, situations, or for specific objects. For example, arachnophobia is fear for spiders, and claustrophobia is a fear for small spaces. There are many different types of anxiety disorders that lead to deviant thoughts, feelings or behavior. Psychologists have long investigated how to overcome fear for non-harmful situations or objects. Exposure therapy is an effective approach for treating anxiety disorders and facilitates an inhibitory learning approach called fear extinction. Fear extinction is defined as a decline in conditioned fear responses (CRs) following non-reinforced exposure to a feared conditioned stimulus (CS). Behavioral evidence indicates that extinction is a form of inhibitory learning: Extinguished fear responses reappear with the passage of time (spontaneous recovery), a shift of context (renewal), and unsignaled presentations of the unconditioned stimulus (reinstatement). Because of this reason: in order to prolong and sustain the inhibition over a long period of time and in the actual real world with all challenging situations and objects present, it is important that the fear extinction holds.

Next to fear extinction, it could be mentioned that in depression situations triggering maladaptive thoughts can be displayed. In psychosis, illusions can be presented, in pain treatment mirror visual feedback can be provided, etc. This is where virtual reality can play a major role.

Virtual reality can create a realistic, but essentially non-harmful training situation for a therapy setting where the therapist and patient with mental health problem work together on gradually increasing the exposure to the feared situation or object.

In order to optimize the therapeutic effect, it is beneficial to keep track of the emotional level of the patient with mental problems. The aim of the exposure therapy is to gradually get familiar / relaxed with a non-harmful but fear-inducing object or situation. Therefore, it is beneficial to personalize the treatment sessions in intensity of the experienced fear. To give an example, for a patient with fear for spiders 402, 602, 702, the size of the spider (image 402 vs 602), the amount of hair (704) of the spider, the proximity of the spider, the colors ... are all variables that can be adapted in the treatment. These variables are best specified in a feature table that drives the content in the virtual environment. Next to visual variables, one can also think about tactile variables, like a tingling sensation of a spider creeping over your hand, or the sound of the spider. Multiple modalities (visual, tactile, auditory, ...) can add characteristics to the content in the 'virtual reality'. The more realistic the content in the virtual reality, the better the therapeutic effect because of transfer effects of the learned (ecologically valid) context to the real world.

fMRI neurofeedback may be applied to learn and regulate emotions. Music may be used to induce emotional responses (i.e., tenderness and anguish trials) and subjects may voluntarily regulate the induced emotions by providing them with real-time information regarding their brain activation. Guided by the color changes of the virtual environment BCI during rtfMRI-NFB, participants may increase in real time, the activity of the septohypothalamic area and the amygdala during the ROI based rtfMRI-NFB, and may successfully evoke distributed patterns of brain activity classified as tenderness and anguish during SVM-based rtfMRI-NFB.

A goal of exposure therapy is to gradually increase comfort with non-harmful but fear-inducing situations or objects. In order to do this, a virtual reality tool can render a controlled training situation where the therapist can guide the patient with mental health problems to cope with the fear-inducing situation. Optimization of the therapeutic effect can be achieved through real-time generation of content that resonates best with the patient's fear level. Examples may possibly be used for exposure therapy.

For other therapies, the Virtual Reality (VR) tool may be applied for different purposes: for example, for substance abuse therapies the VR system is used as a stimulus to induce levels of craving in a controlled, non-harmful manner. The transfer of learned skills from the therapy environment to the real world are biggest when the stimulation content is immersive, and specifically personalized to the relevance for an individual patient.

Taking anxiety as an example, the personalization of the therapy can be done based on the subjective fear level (through questionnaires like a Visual Analogue Scale to rate the fear level).

There are however a few drawbacks / problems / disadvantages of applying the subjective fear level as the driver of the feature table:
- One challenge is that it is rather impractical to track subjective feelings continuously over time. The attention of the patient should be with the gradual relaxation during fearful content, and preferably not with the continuous judgement of the subjective fear level.
- Another disadvantage is the fact that the -patient cannot easily provide an objective rating of the fear. This can lead to an overshoot in the content of the virtual reality, i.e. the patient is being exposed to a too fearful situation or object whereby the therapeutic effect shuts down and the patient closes up for further exposure.

For other disorders, a similar approach to personalization encounters the same issue of subjectivity of the patient's response to the VR content.

Examples may potentially overcome the above-mentioned shortcomings by providing an immersive personalized stimulation device and method to help therapy making use of real-time brain signals.

Examples may provide an approach consisting of a method and a system to optimize virtual really therapy for individuals suffering from psychiatric or mental diseases, such as anxiety. Optimization of the therapy is achieved by means of an in-bore close loop stimulation system and method. The stimulation system readapts real-time the immersive multi-sensory virtual-reality stimulation in order to provide a personalized system with real-time input from fMRI.

Concretely examples may provide a stimulation system and a method that allows real time adaption of the stimulation conditions (parameters and features) according to the brain activity detected.

Thus, examples may provide for a stimulation system that is a closed loop system where the real-time measurement of brain activity is employed by a controller to dynamically and parametrically adapt the variables in the feature table, which drive the content in the immersive multisensory personalized virtual environment. Examples may include one or more of the following features:
- A functional brain activation monitoring unit (e.g., fMRI, EEG, fNIRS, OPM, MEG, etc...)
- A virtual reality therapy unit (medical instrument 100, 300):
   - Device to provide virtual content 106 to the user; i.e., headset or a 3D television that can generate immersive content with e.g., depth cues (either MR compatible or making use of a mirror to project into the MR bore).
   - Device to provide sensory stimulus or combination of stimuli multisensory hardware (eg a tactile stimulation device 106'" to generate a tingling spider sensation).
- Optional extension towards hardware that is able to stimulate the motor system preferably integrated with sensory stimulation).
- An actuator unit:
   - to collect the information of the monitoring unit
   - to analyze the information gathered
   - to send the information to the virtual really unit

Thus, examples may comprise a system and method where a virtual reality unit i.e., 3D display for rendering an immersive virtual environment wherein the content to be exposed to a psychiatric patient, is defined by one or more variables in a feature table (like e.g. the proximity, size, ... of an emotional object or a situation) and a brain recording and processing unit (e.g. real-time fMRI, EEG, fNIRS, ...).

The system may include other monitoring units such as sensory units to monitor physiological parameters such as heart rate, respiration rate, skin conductance, eye movement and pupil width.

Below several examples are described, where the description of the technical features of the example has as main application for generic disorders. In further examples, details of the system - define to a specified disorder.

### Example 1: Closed loop system: real time adaptation of virtual reality stimulation based on brain activity measurements

Real-time measurement of the brain activity of a patient is used to adapt the virtual reality stimulation presented to the patient.

The virtual reality stimulation system comprises a stimulus or set of sensory stimuli that preferably comprise at least a visual stimulus.

The visual stimulus can be created by a virtual reality headset or by any other display.

In a preferred embodiment, the display or VR headset renders 3D image information - as will be described in more detail in embodiment 2.

In a more advanced system, the virtual reality stimulation may include other kind of stimuli for example audio or tactile stimulation - using standard components such as loudspeakers and tactile actuators such as vibrating devices, tapping devices etc.

Brain activity monitoring unit: Determination of the brain activity of the patient may be done real-time by one of the following systems:
- An fMRI system, where the subject is present in the bore of the MRI machine such as to facilitate a functional scan of (at least a specified region of) the brain of the subject
- An EEG system, where the subject wears a cap with one or a multitude of electrodes that measure the electrical activity of the brain of the subject
- An OPM, where the subject wears a cap with one of a multitude of optically-pumped magnetometers measure the electrical activity of the brain of the subject
- A fNIRS system, where the subject wears a cap with one or a multitude of light emitting diodes that optically measure the blood flow in the brain of a subj ect

The workflow or method may comprise one or more of the following steps:
- Step 1: the subject is arranged such that brain activity can be measured in real time
- Step 2: a baseline measurement of the brain activity of the subject is established. This baseline could include non-emotional stimuli.
- Step 3: a first therapeutic stimulus is presented to the subject and the changes of brain activity relative to the baseline are measured
- Step 4: based upon this change in brain activity, at least one feature of the stimulus (such as contrast, size, velocity, color) is parametrically varied
- Step 5: the new stimulus is presented to the subject and the changes of brain activity relative to the baseline (or relative to that measured as a response to the previous stimulus) is measured
- Step 6: Steps 4 and 5 are repeated for a series of stimuli
- Step 7: once the desired series of stimuli are completed (evoking the desired brain activity), the subject is removed from the system

In particular, step 4 of the process may adapt to the specific disorder of the patient, and will be described in detail below. However, in many cases the following aspects will be considered:
In the case that the measured brain activity response in the brain region of interest (one or more volumes selected from within the excitation region of interest) is in the direction expected (below the second threshold and moving towards the first threshold), the feature may be adapted as to slightly strengthen the effect in the new stimuli. This represents a case where the feedback is positive.
- In the case that the measured brain activity response in the brain region of interest is neutral (or not in the direction expected), the feature may be adapted as to strongly strengthen the effect in the new stimuli. This represents a case where the feedback is neutral.
- In the case that the measured brain activity response in the brain region of interest is neutral (or not in the direction expected) AND a strong response above the second threshold is measured in an unexpected region of the brain the feature will be adapted as to weaken the effect in the new stimuli. This represents a case where the feedback is negative (i.e., the subject is responding negatively to the stimulus). If this situation persists, the series of stimuli may be aborted.

### Example 2: Presentation of the fear object using 3D depth cues

As was described above, one of the major factors in manipulation of the virtual visual stimuli is the perceived distance of the object from the subject.

For this reason, in another example the system comprises a 3D display comprising e.g. lenses or using polarization glasses. In either case the real time brain activity is used to modify one or more features of the 3D image - and in particular a feature related to a 3D depth cue. Such features are e.g. 3D depth of the image, the position of the image relative to the plane of the display and other binocular cues. In such a manner it is possible to e.g., manipulate the position of the image closer or further away from the user as a result of the measured skin conductance of the user. In order to achieve best effect, the 3D display should be configured such that it displays a maximum 3D depth and more in particular the ability to render the object far in front of the screen. Such configuration can be realized by a specific choice of lens pitch, lens slope, pixel size and image rendering on the pixels.

In the case that a 3D display with lenses is used in combination with fMRI, the display is most advantageously viewed by the subject using a mirror angled at 45 degrees placed above the subject's head in the bore of the scanner. As a consequence, the image on the actual display must be inverted so as to render the image correctly to the subject (i.e., with objects reacting to gravity in the correct manner).

### Example 3: Multisensory rhythmically synchronous stimulation

In this embodiment the stimulation comprises a combination of stimuli, such as visual, auditory, and tactile stimuli in a specific rhythmically synchronous way.

There is evidence that rhythmical synchronous combination of sensory modalities helps a patient to focus on the sensory stimulation. Inspiration for the use of rhythmically synchronous signals for this invention came from fundamental neuroscience experiments by one of the authors of this ID proposal on the behavioral effects of signal congruency across sensory brain modalities. Heteromodal congruency is an efficient means for the brain to identify signal relevance in the bombardment of sensory signals. There is a machinery of neurons especially devoted to combine perceptual functions across sensory modalities. These neurons play an important role in perceptual control to influence conscious experiences. Signal congruency, then, facilitates multimodal mechanisms of voluntary perceptual control, since there is more support for a particular percept when there is information from another sensory modality that is congruent with it. Indeed, experiments revealed that the capacity to voluntarily select one of two competing percepts is greatly enhanced when there is such congruency (in some observers over 400% increase in control over perception. In addition, internal Philips' research has shown that rhythmically synchronous visual and auditory stimulation did positively influence the breathing rate. Moreover, the patient is more relaxed because they can increasingly focus on the stimulation instead of on their stress [indeed multisensory stimulation has been shown to lower blood pressure. All in all, Multisensory rhythmically synchronous stimulation increases the immersiveness of the stimulation thereby better penetrating the perceptual brain. For example, a multisensory system could mimic walking of a spider.

### Example 4: Stimulation device for anxiety disorder

In this example an adaptation of the stimulation system to enable parametrically modify stimuli suitable for subjects with anxiety disorders is considered. Here, a potential goal of the process is to monitor the activity of the limbic system after the presentation of the anxiety inducing stimulus and to achieve a state where either:
- The activity reduces as the same stimulus is presented and/or
- The activity remains constant or even reduces as the intensity of the stimulus becomes higher (i.e., should induce more anxiety)

To illustrate the operation principle, a case of anxiety (phobia) induced by the presence of a visual stimulus (in this case a spider) is considered.
Here, the virtual presence of the fear object (e.g., a spider 402 in a cage 404) is adapted based on a measured real time brain activity of the subject. In this case the feature which is adapted is the perceived distance of the object from the subject (see embodiment below for details of how this is achieved using 3D object rendering).
Operation of the system is as follows
- Step 1: The system starts with the presentation of the fear object at a first distance (of e.g., 1 meter) from the user: this is stimulus 1
- Step 2: The activity of the limbic system after the presentation of stimulus 1 is monitored: it is expected that the activity will increase
- If the increase of activity is higher than expected, the stimulus is considered to induce too much anxiety and a new stimulus is considered at a second distance which exceeds the first distance (e.g., 2 meters)
- If the increase of activity is lower than expected, the stimulus is considered not to induce too much anxiety and a new stimulus is considered at a second distance which is less than the first distance (e.g., 0.8 meters)
- If the increase of activity is roughly as expected, the stimulus is considered to induce an acceptable level of anxiety and a repeating of the first stimulus is initiated. Depending upon the new activity level the stimulus will be then brought nearer or moved further away as described above.
- Step 3: Steps 1 and 2 are repeated until the desired reduction in limbic activity is achieved.

This calculated parameter will now determine the value from one or more variables in a feature table. In this example, the variable in the feature table could be "the proximity to the feared object". Other features in the table could be the size of the feared object, or the amount of hair of the spider etc.

### Example 5: Stimulation device for mental disorders in general such as addictions, pain, obsessive-compulsive disorder, and etc.

The present example could also be used for other applications such as for mental disorders in general such as addictions, subjective pain perception, obsessive compulsive disorder, other phobias, PTSD, sleep disorders, eating disorders, bipolar disorders, schizophrenia, etc. The stimuli of combination of stimuli can be (re)adapted specifically for the mental disorder at hand.

### Example 6: Presentation of the fear object is adapted based on physiological measures like heart rate and respiration rate

This example is very similar to previous embodiments but here we do NOT use real-time signals from the brain scanner. Instead, real-time signals from physiological measures such as heart rate and respiration rate collected via an MR compatible sensor are used.

### Example 7: Inclusion of the motor system

This example includes involvement of the motor system preferably integrated with the sensory system. Here one can think of a motor action of the patient where she/he attempts to hit a spider by moving the hand. Here the stimulation device requires monitoring of the hand which can be done with conventional methods. In a related embodiment, the patient in the brain scanner performs an exercise such as cycling.

### Example 8: Inclusion of smell

This embodiment includes involvement of smell depending on the distance of an object to be mimicked.

### Example 9: Inclusion of neurofeedback

This embodiment includes involvement of neurofeedback depending on the voluntary neuronal activity evoked by the subject.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical instrument
- 102: subject
- 104: subject support
- 106: virtual reality system - virtual reality glasses
- 106': virtual reality system - olfactory stimulus generator
- 106": virtual reality system - headphones
- 106''': virtual reality system - tactile feedback system
- 106'''': virtual reality system - temperature control system (heater and or cooling system)
- 110: activity measurement system
- 112: computer
- 114: computational system
- 116: hardware interface
- 118: user interface
- 120: memory
- 130: machine executable instructions
- 132: simulation model
- 134: simulation data
- 136: brain activity data
- 138: neural activity metric
- 140: model parameter
- 142: initial value
- 144: first predetermined value
- 146: second predetermined value
- 200: set the model parameter to an initial value
- 202: generate the simulation data using the simulation model
- 204: render the simulation data using the virtual reality system
- 206: measure the brain activity data using the activity measurement system
- 208: calculate a neural activity metric descriptive of neural activity of the subject from the brain activity data
- 210: increase the model parameter to adjust the simulation model if the neural activity metric is below a first predetermined value
- 212: decrease the model parameter to adjust the simulation model if the neural activity metric is above a second predetermined value
- 300: medical instrument
- 301: optional EEG system
- 302: magnetic resonance imaging system
- 304: magnet
- 306: bore of magnet
- 308: imaging zone
- 309: excitation region of interest
- 310: magnetic field gradient coils
- 312: magnetic field gradient coil power supply
- 314: radio-frequency coil
- 316: transceiver
- 320: pulse sequence commands
- 322: k-space data
- 324: functional magnetic resonance image
- 326: optional EEG data
- 328: control algorithm
- 400: three-dimensional image
- 402: spider (far away)
- 404: cage
- 500: three-dimensional image
- 402: spider (far away)
- 600: three-dimensional image
- 602: friendly spider (close)
- 700: three-dimensional image
- 702: spider (close)
- 704: leg hair

## Claims

1. A medical instrument (100, 300) comprising:
- an activity measurement system (110, 301, 302) configured for measuring brain activity data (136) from a subject (102);
- a memory (120) storing machine executable instructions (130) and storing a simulation model (132), wherein the simulation model is configured to generating simulation data (134), wherein the simulation model is configured for modifying the simulation data using a model parameter (140);
- a virtual reality system (106, 106', 106", 106"', 106"") configured for rendering the simulation data; and
- a computational system (114) for controlling the medical instrument, wherein execution of the machine executable instructions causes the computational system to set (200) the model parameter to an initial value (142), wherein execution of the machine executable instructions further causes the computational system to repeatedly:
- generate (202) the simulation data using the simulation model;
- render (204) the simulation data using the virtual reality system;
- measure (206) the brain activity data using the activity measurement system;
- calculate (208) a neural activity metric (138) descriptive of neural activity of the subject from the brain activity data;
- increase (210) the model parameter to adjust the simulation model if the neural activity metric is below a first predetermined value (144); and
- decrease (212) the model parameter to adjust the simulation model if the neural activity metric is above a second predetermined value (146).

2. The medical system of claim 1, wherein the brain activity measurement system comprises a magnetic resonance imaging system (302), wherein the memory further contains pulse sequence commands (320) configured to acquire k-space data descriptive of an excitation region of interest of the subject according to a functional magnetic resonance imaging protocol, wherein measuring the brain activity data using the activity measurement system comprises controlling the magnetic resonance imaging system to acquire the k-space data.

3. The medical instrument of claim 2, wherein the magnetic resonance imaging protocol is any one of the following a blood oxygenation level dependent functional magnetic resonance imaging protocol and configured for measuring neural activity within one or more volumes selected within the excitation region of interest.

4. The medical instrument of claim 3, wherein the one or more volumes comprises any one of the following: the amygdala, the ventromedial prefrontal cortex, the fronto-parietal network, the hippocampus, and combinations thereof.

5. The medical instrument of any one of the preceding claims, wherein the activity measurement system comprises any one of the following: a magnetoencephalography system, an electroencephalography system, an optically pumped magnetometer magnetoencephalography brain measurement system, a functional near-infrared spectroscopy system, an eye dilation measurement system, a heart rate measurement system, a respiration rate system, a subject motion detection system, and combinations thereof.

6. The medical instrument of any one of the preceding claims,
wherein the memory further contains a control algorithm (328) configured for outputting the model parameter in response to receiving the neural activity metric, the first predetermined value, and the second predetermined value, wherein execution of the machine executable instructions further causes the computational system to determine the model parameter by inputting the neural activity metric, the first predetermined value, and the second predetermined value into the control algorithm.

7. The medical instrument of claim 6, wherein the control algorithm is implemented as any one of the following: a lookup table, a multi-dimensional lookup table, a neural network, and a feedback loop.

8. The medical instrument of any one of the preceding claims, wherein the virtual reality system comprises any one of the following: a visual display (106), a three-dimensional display (106), a virtual reality headset (106) or an augmented reality headset, a headphone (106"), an audio speaker, a subwoofer, a tactile feedback system (106"'), a heater (106""), a cooler (106""), an instruction display for providing manual tactile feedback instructions, an olfactory stimulus generator (106'), and combinations thereof.

9. The medical instrument of any one of the preceding claims, wherein the virtual reality system is configured for rendering multiple sensory stimuli using rhythmically synchronous stimulation.

10. The medical instrument of any one of the preceding claims, wherein the simulation module is configured such that an increase in the model parameter induces an initial increase in the neural activity metric.

11. The medical instrument of any one of the preceding claims, wherein the decrease of the model parameter if the neural activity metric is above the second predetermined value is a function of the velocity of the neural activity metric when it exceeded the second predetermined value.

12. The medical instrument of claim 11, wherein the value of the model parameter decreases as the velocity of the neural activity metric increases when it exceeds the second parameter value.

13. A computer program comprising machine executable instructions (130) for execution by a computational system (114) controlling a medical instrument (100, 300), wherein the medical instrument comprises an activity measurement system (110, 301, 302) configured for measuring brain activity data (136) from a subject (102), wherein the medical instrument further comprises a virtual reality system (106, 106', 106", 106'", 106"") configured for rendering simulation data to the subject, wherein the medical instrument further comprises a memory (120) storing a simulation model (132), wherein the simulation model is configured to generate simulation data (134), wherein the simulation model is configured for modifying generation of the simulation data using a model parameter (140); wherein execution of the machine executable instructions causes the computational system to set (200) the model parameter to an initial value (142), wherein execution of the machine executable instructions further causes the computational system to repeatedly:
- generate (202) the simulation data using the simulation model;
- render (204) simulation data using the virtual reality system;
- measure (206) the brain activity data using the activity measurement system;
- calculate (208) a neural activity metric (138) descriptive of neural activity of the subject from the brain activity data;
- increase (210) the model parameter to adjust the simulation model if the neural activity metric is below a first predetermined value (144); and
- decrease (212) the model parameter to adjust the simulation model if the neural activity metric is above a second predetermined value (146).

14. A method of controlling a medical instrument (100, 300), wherein the medical instrument comprises an activity measurement system (110, 301, 302) configured for measuring brain activity data (136) from a subject (102), wherein the medical instrument further comprises a virtual reality system (106, 106', 106", 106"', 106"") configured for rendering simulation data to the subject, wherein the medical instrument further comprises a memory (120) storing a simulation model (132), wherein the simulation model is configured to generate simulation data (134), wherein the simulation model is configured for modifying the simulation data using a model parameter (140); wherein the method comprises setting (200) the model parameter to an initial value;
wherein the method comprises repeatedly:
- generating (202) the simulation data using the simulation model;
- rendering (204) simulation data using the virtual reality system;
- measuring (206) the brain activity data using the activity measurement system;
- calculating (208) a neural activity metric (138) descriptive of neural activity of the subject from the brain activity data;
- increasing (210) the model parameter to adjust the simulation model if the neural activity metric is below a first predetermined value (144); and
- decreasing (212) the model parameter to adjust the simulation model if the neural activity metric is above a second predetermined value (146).

15. The method of claim 14, wherein the method further comprises selecting the simulation model such that rendering the simulation data using the virtual reality system evokes an emotional or psychological response from the subject that is initially increased by an increase in the model parameter.
